# EUROPÄISCHE PATENTSCHRIFT

(11) **EP 3 939 422 B1**
(45) Veröffentlichungstag und Bekanntmachung des Hinweises auf die Patenterteilung: **04.09.2024**
(21) Anmeldenummer: 21185099.5
(22) Anmeldetag: 12.07.2021
(51) Int. Cl.: A01K 63/00, A01K 63/06, A01K 1/00

(54) **(AQUA-)TERRARIUM**
(AQUA-) TERRARIUM
(AQUA)TERRARIUM

(30) Priorität: 14.07.2020 DE 102020118579
(43) Veröffentlichungstag der Anmeldung: 19.01.2022
(73) Patentinhaber: OASE Holding U.K. Limited, Southampton SO50 9PD (GB)
(72) Erfinder: Muck, Thorsten, 49479 Ibbenbüren (DE)
(74) Vertreter: Engelmann, Kristiana

(56) Entgegenhaltungen:
- WO-A1-2015/012698
- US-A1- 2008 014 857
- US-A1- 2013 305 605
- US-A1- 2018 279 563

## Beschreibung

Die Erfindung betrifft ein Terrarium oder Aquaterrarium, insbesondere für ektotherme Tiere, mit einem als Lebensraum dienenden Behältnis.

Terrarien sowie Aquaterrarien sind Vivarien zur Haltung verschiedener, insbesondere exotischer Tiere und/oder Pflanzen. Als Lebensraum dient dabei ein zumindest bereichsweise einsehbares Behältnis, in dem ein Stück Land, d.h. ein Stück begehbar oder bepflanzbarer Boden, eingerichtet werden kann. Man spricht von Terrarien, wenn im fertig eingerichteten Zustand des Vivariums der Landanteil und/oder der Luftraum überwiegt. Hingegen spricht man von Aquaterrarien, wenn der Wasseranteil dominiert.

Bei der Gestaltung eines (Aqua-)Terrariums wird in der Regel versucht, das natürliche Habitat der zu haltenden Art so gut wie möglich nachzubilden, wobei die Schaffung geeigneter klimatischer Bedingungen - wie Temperatur, Luftfeuchte und/oder Lichtverhältnisse - einen Schwerpunkt bildet. Insbesondere bei der Haltung von ektothermen Tieren, wie beispielsweise Reptilien, die im Gegensatz zu Säugetieren keine eigene Körperwärme produzieren und zur Regulation ihrer Körpertemperatur auf externe Wärme- oder Kältequellen angewiesen sind, ist ein adäquates Temperaturmanagement essenziell. Andernfalls können Unterkühlung oder Überhitzung zu Erkrankungen der Tiere führen.

Aus dem Stand der Technik ist es bekannt, die Temperatur in (Aqua-)Terrarien etwa durch Wärmelampen oder Heizdrähte zu regulieren, die auf einen Punkt innerhalb des Behältnisses fokussiert sind. Ektotherme Tiere können sich zum Aufwärmen in die Wärmestrahlungszone des jeweiligen Heizmittels bewegen. In der Regel werden Wärmelampen und Heizdrähte innerhalb des Behältnisses angeordnet, wodurch für die Tiere ein Verletzungsrisiko durch direkten Kontakt besteht. Hinzu kommt, dass sich mit Wärmelampen oder Heizdrähten Temperaturen oder Temperaturverteilungen innerhalb des (Aqua-)Terrariums nur schwer einstellen lassen. Auch sind Wärmelampen und Heizdrähte nicht mit einem Vernebelungssystem zur Regulierung der Luftfeuchtigkeit kompatibel, da dabei das Risiko eines Stromschlages nicht ausgeschlossen werden kann.

Aus der US 2018/0279563 A1 ist ein System zur Simulation der Umweltbedingungen eines Lebensraumes mit den vorstehenden Nachteilen bekannt.

Aufgabe der vorliegenden Erfindung ist es daher, eine verbesserte Möglichkeit zur Regulierung der klimatischen Bedingungen eines (Aqua-)Terrariums bereitzustellen, die zumindest eine Temperaturregulierung umfasst.

Diese Aufgabe wird gelöst durch ein (Aqua-)Terrarium mit den Merkmalen des Anspruchs 1.

Das erfindungsgemäße (Aqua-)Terrarium zeichnet sich durch eine Klimatisierungsvorrichtung aus, die eine Strömungsmaschine, vorzugsweise einen Lüfter, sowie einen elektrothermischen Wandler, insbesondere ein Peltier-Element, umfasst. Hierbei saugt die Klimatisierungsvorrichtung Luft aus dem Behältnis an, konditioniert die angesaugte Luft durch Entlangführen an einer ersten Seite des elektrothermischen Wandlers und befördert die konditionierte Luft zurück in das Behältnis. Die Regulierung der Temperatur erfolgt somit zumindest zum Teil durch eine Konditionierung eines zirkulierenden Luftstroms. Der als Wärme- und/oder Kältequelle dienende thermoelektrische Wandler muss nicht direkt in dem Behältnis angeordnet oder auf das Behältnis gerichtet sein, was das Risiko von Verletzungen etwa durch Wärme-/Kältespitzen oder direkten Kontakt verringert. Ferner kann auf Wärmelampen und/oder Heizdrähte gänzlich verzichtet werden. Falls unter Umständen Wärmelampen und/oder Heizdrähte dennoch unterstützend eingesetzt werden, können diese kleiner dimensioniert und/oder mit größerem Abstand, z.B. in nicht zugänglichen Bereichen des (Aqua-)Terrariums, positioniert werden.

Die Strömungsmaschine erzeugt einen erzwungenen Luftstrom, der die Luft an der ersten Seite des elektrothermischen Wandlers entlangführt. Der elektrothermische Wandler ist in einfacher Weise entsprechend den jeweiligen Anforderungen ansteuerbar. Durch Anlegen einer Spannung an die beiden Kontakte des elektrothermischen Wandlers, in der Regel einer Gleichspannung, wird dessen erste Seite beispielsweise heiß, wobei die daran entlanggeführte Luft erhitzt wird. Durch einfache Umkehr der Stromrichtung wird vom Heiz- auf den Kühlmodus umgeschaltet. Die erste Seite des Wandlers wird kalt und kühlt die daran entlanggeführte Luft.

Der elektrothermische Wandler ist außerhalb des Behältnisses angeordnet. Dadurch wird die Verletzungsgefahr etwa durch Wärme-/Kältespitzen oder direkten Kontakt weiter verringert. Der durch die Strömungsmaschine erzeugte Luftstrom führt in diesem Fall aus dem Behältnis raus, zum Zwecke der Konditionierung an dem elektrothermischen Wandler entlang und anschließend zurück in das Behältnis hinein. Bevorzugt ist der elektrothermische Wandler hinter einer der den Lebensraum begrenzenden Wände des Behältnisses, bevorzugt einer nicht einsehbaren Rückwand, angeordnet.

Alternativ oder zusätzlich zu der Anordnung des elektrothermischen Wandlers außerhalb des Behältnisses ist die Strömungsmaschine außerhalb des Behältnisses, bevorzugt hinter einer der den Lebensraum begrenzenden Wände des Behältnisses, bevorzugt einer nicht einsehbaren Rückwand, angeordnet. Auf diese Weise wird einer Verletzungsgefahr entgegengewirkt, da sich keine beweglichen Teile der Strömungsmaschine innerhalb des Behältnisses befinden.

In einer weiteren bevorzugten Ausgestaltung der Erfindung ist zumindest die erste Seite des elektrothermischen Wandlers rechtwinklig zu einer nächstliegenden, den Lebensraum begrenzenden Wand des Behältnisses, insbesondere einer Seiten- oder Rückwand, angeordnet. Dadurch ist die erste Seite nicht direkt auf die Wand gerichtet, so dass diese beispielsweise im Heizmodus nicht zu stark erwärmt wird, was sonst beim Berühren der Wand zu Verletzungen führen könnte. Die nächstliegende Wand zum elektrothermischen Wandler ist diejenige Wand, die den geringsten Abstand zum Wandler aufweist, z.B. eine Seiten- oder Rückwand, hinter der der Wandler angeordnet ist. Die nächstliegende Wand kann auch ein Boden oder eine Decke des Behältnisses sein.

In einer weiteren bevorzugten Ausgestaltung der Erfindung weist die Klimatisierungsvorrichtung einen Luftkanal auf, der zumindest teilweise, vorzugsweise vollständig, außerhalb des Behältnisses verläuft, wobei die erste Seite des elektrothermischen Wandlers oder ein mit der ersten Seite thermisch leitend verbundener Kühlkörper einen Teil der Wandung des Luftkanals ausbildet. Auf diese Weise wird der Luftstrom optimal entlang des elektrothermischen Wandlers geführt. Darüber hinaus wird eine kompakte Bauweise der Klimatisierungsvorrichtung ermöglicht. Bevorzugt weist die erste Seite des elektrothermischen Wandlers einen Kühlkörper auf, z.B. in Form eines Aufsatzes mit Kühlrippen, der in den Luftkanal hineinragt. Durch den Einsatz von beispielsweise Kühlrippen wird die Oberfläche der ersten Seite des elektrothermischen Wandlers vergrößert, wodurch die Luft effizienter konditioniert wird. Insbesondere ist der Luftkanal derart ausgebildet, dass zumindest in einem Abschnitt des Luftkanals, in dem sich der elektrothermische Wandler oder ein mit dessen erster Seite thermisch leitend verbundener Kühlkörper befindet, eine Wandung des Luftkanals die nächstliegende Wand des Behältnisses, beispielsweise eine Rückwand des Behältnisses, nicht berührt. Auf diese Weise wird eine noch bessere thermische Trennung zwischen der Wärme-/Kältequelle und dem Behältnis erreicht und so das Risiko von Verletzungen etwa durch Wärme-/Kältespitzen oder direkten Kontakt weiter verringert.

In einer weiteren bevorzugten Ausgestaltung der Erfindung weist die Klimaanlage zum Behältnis hin eine Einlassöffnung und eine Auslassöffnung auf, wobei die Einlassöffnung in einem unteren Bereich des Behältnisses und die Auslassöffnung in einem oberen Bereich des Behältnisses angeordnet ist. Auf diese Weise kann eine optimale Zirkulation der Luft erreicht werden. Ferner lässt sich auf diese Weise ein Temperaturgradient entlang einer Vertikalen schaffen, so dass insbesondere ektotherme Tiere bei entsprechend eingerichtetem (Aqua-)Terrarium ihre Körpertemperatur durch Aufsuchen einer bestimmten Höhenlage regulieren können. Beispielsweise können wärmere Temperaturen im oberen Beriech hin zu tieferen Temperaturen im unteren Bereich des Behältnisses erzeugt werden.

Dabei wird als der obere Bereich des Behältnisses eine obere Hälfte, insbesondere ein oberes Drittel, bevorzugt ein oberes Viertel, des Behältnisses bezogen auf die gesamte Höhe des Behältnisses bezeichnet. Ferner wird als der untere Bereich des Behältnisses eine untere Hälfte, insbesondere ein unteres Drittel, bevorzugt ein unteres Viertel, des Behältnisses bezogen auf die gesamte Höhe des Behältnisses bezeichnet. Insbesondere werden bei einem Terrarium mit Wasseranteil oder einem Aquaterrarium der untere Bereich und der obere Bereich nicht auf die Gesamthöhe des Behältnisses, sondern auf eine um einen Wasserspiegel innerhalb des Behältnisses verkürzte Höhe, z.B. eine Luftraumhöhe, bezogen. Vorzugsweise ist die Einlassöffnung oberhalb des ein Stück Land in dem Behältnis bildenden Substrat oder eines Wasserspiegels angeordnet. Vorzugsweise sind sowohl die Einlassöffnung als auch die Auslassöffnung an einer einzigen Seiten- oder Rückwand des Behältnisses angeordnet.

In einer weiteren bevorzugten Ausgestaltung der Erfindung weist die Klimatisierungsvorrichtung einen Wärmeabfuhrkanal auf, der außerhalb des Behältnisses verläuft, wobei eine zweite Seite des elektrothermischen Wandlers, die der ersten Seite des elektrothermischen Wandlers gegenüberliegt, oder ein mit der zweiten Seite thermisch leitend verbundener Abluft-Kühlkörper einen Teil der Wandung des Abluftkanals ausbildet. Auf diese Weise kann etwa im Kühlmodus der Klimatisierungsvorrichtung die Abwärme, die an der zweiten Seite des elektrothermischen Wandlers entsteht, optimal in die Umgebung abgeführt werden. Ist der elektrothermische Wandler zudem rechtwinklig zu einer nächstliegenden Wand angeordnet, sind weder seine erste noch seine zweite Seite direkt auf die Wand gerichtet, was einem Aufheizen oder Abkühlen der Wand entgegenwirkt. Vorzugsweise ist der Wärmeabfuhrkanal mit einer eigenen Strömungsmaschine ausgestattet, die einen erzwungenen Abluftstrom erzeugt und so die Wärmeabfuhr verbessert.

Bevorzugt weist die zweite Seite des elektrothermischen Wandlers einen Abluft-Kühlkörper auf, z.B. in Form eines Aufsatzes mit Kühlrippen, der in den Wärmeabluftkanal hineinragt. Dadurch wird die Oberfläche der zweiten Seite des elektrothermischen Wandlers vergrößert, wodurch die Wärme effizienter an die Abluft abgegeben werden kann.

In einer weiteren vorteilhaften Ausgestaltung der Erfindung weist das (Aqua- )Terrarium eine Steuer- und Regelungseinheit auf, die mit zumindest einem Temperatursensor datenübertragend verbunden ist, und die Strömungsmaschine und/oder den elektrothermischen Wandler regelt. Dadurch kann eine automatisierte Temperaturregelung innerhalb des Behältnisses erfolgen, wodurch das Risiko einer Überhitzung oder Unterkühlung innerhalb des (Aqua-)Terrariums verringert wird. So kann der Steuer- und Regelungseinheit ein Soll-Wert oder ein Soll-Wertebereich vorgegeben werden. Als Entscheidungskriterium über eine Ansteuerung der Strömungsmaschine und/oder des elektrothermische Wandlers kann ein Soll-Ist-Vergleich der vorgegeben Soll-Werte mit innerhalb des Behältnisses durch den Temperatursensor aufgezeichneten Ist-Werten dienen.

In einer weiteren bevorzugten Ausgestaltung der Erfindung ist die Steuer- und Regelungseinheit mit zumindest einem Luftfeuchtigkeitssensor datenübertragend verbunden, wobei die Steuer- und Regelungseinheit einen Vernebler der Klimatisierungsvorrichtung regelt, insbesondere einen Ultraschall-Vernebler, mit dem Wasser aus einem Wasserreservoir der Klimatisierungsvorrichtung vernebelbar und zur Erhöhung der Luftfeuchtigkeit in das Behältnis abgebbar ist. Die erfindungsgemäße Klimatisierungsvorrichtung lässt sich im Gegensatz zu Wärmelampen ohne Sicherheitsbedenken mit einem Vernebelungssystem kombinieren, wodurch das Klima im (Aqua-)Terrarium in höherem Umfang reguliert und so das natürliche Habitat der zu haltenden Art noch besser nachgebildet werden kann.

Vorzugsweise ist die Steuer- und Regelungseinheit zumindest mit einem ersten Sensor, der zumindest als Temperatursensor ausgebildet ist, und einem zweiten Sensor, der zumindest als Temperatursensor ausgebildet ist, datenübertragend verbunden, wobei der erste Sensor in einem oberen Bereich des Behältnisses angeordnet ist und der zweite Sensor in einem unteren Bereich des Behältnisses angeordnet ist. Dadurch kann entlang einer Vertikalen ein Temperaturgradient gemessen und zur verbesserten Regelung zumindest der Strömungsmaschine und/oder des elektrothermischen Wandlers verwendet werden. Vorzugsweise ist der erste Sensor im Wesentlichen auf Höhe oder oberhalb der Auslassöffnung angeordnet. Bevorzugt ist der zweite Sensor unterhalb oder im Wesentlichen auf Höhe der Einlassöffnung der Klimatisierungsvorrichtung angeordnet.

In einer weiteren bevorzugten Ausgestaltung der Erfindung ist die Steuer- und Regelungseinheit derart eingerichtet, dass - insbesondere in Abhängigkeit des zu haltenden Tieres - eine vorprogrammierte Parameterkonfiguration von klimataspezifischen Soll-Werten/-Wertebereichen auswählbar ist, die zumindest eine Soll-Temperatur, bevorzugt einen Soll-Temperaturbereich, umfasst. Einem Halter ist es auf diese Weise möglich, kurzerhand ein optimales Klima im (Aqua-)Terrarium für sein Tier einzustellen. Dazu muss er lediglich das für das jeweilige Tier vorgesehene oder passende Programm auswählen. Insbesondere wird eine Auswahl von Programmen bereitgestellt, wobei die Parameterkonfiguration eines jeweiligen Programms auf ein bestimmtes Biom abgestimmt ist bzw. einem bestimmten Biom entspricht, das für ein oder mehrere zu haltende Tiere einen natürlichen Lebensraum darstellt. Da hierdurch das Risiko von Fehleinstellungen und daraus resultierenden Überhitzungen oder Unterkühlungen minimiert wird, ist ein derartiges (Aqua-)Terrarium besonders für Laien geeignet.

Vorzugsweise umfassen die klimataspezifischen Soll-Werte/-Wertebereich auch Vorgaben zur Luftfeuchtigkeit, mit denen beispielsweise ein Vernebelungssystem angesteuert werden kann.

Insbesondere umfasst die Klimatisierungsvorrichtung weitere Systeme zur Beeinflussung bzw. Simulation weiterer Klimaelemente und/oder Gegebenheiten des natürlichen Habitats einer zu haltenden Art wie einer Bodenheizung zur unterstützenden Temperaturregulierung, einem Beregnungssystem, einem Beleuchtungs- bzw. Bestrahlungssystem (z.B. weiße Leuchten, RGB-Leuchten und/oder UV-/IR-Lampen) und/oder zumindest einem Lautsprecher zur Imitation einer natürlichen akustischen Umgebung. Bevorzugt ist die Steuer- und Regelungseinheit zur Regelung zumindest eines dieser Systeme ausgebildet, insbesondere wobei eine vorprogrammierte Parameterkonfiguration entsprechende klimataspezifische Soll-Werte umfasst, z.B. Beregnungsintervalle, Beleuchtungsintervalle/-stärken und/oder zugewiesene Audiodateien.

Besonders bevorzugt ist die Steuer- und Regelungseinheit über eine auf einer Datenverarbeitungsanlage, z.B. einem Heimcomputer, einem Laptop, einem Tablet und/oder einem Smartphone, laufende App steuerbar.

Insgesamt wird mit der vorliegenden Erfindung ein (Aqua-)Terrarium bereitgestellt, bei dem eine verbesserte Regulierung der klimatischen Bedingungen im Behältnis erreicht wird, insbesondere automatisiert und auf das jeweilige zu haltende Tier abgestimmt. Dabei wird zumindest eine Temperatur oder ein Temperaturgradient beeinflusst. In weiterführenden Ausgestaltungen der Erfindung wird ein natürliches Habitat in besonders hohem Maße imitiert, indem ggf. ferner Luftfeuchte, Beregnung, Beleuchtung und/oder eine akustische Umgebung gesteuert werden.

Es wird ausdrücklich darauf hingewiesen, dass die vorstehend erläuterten Ausgestaltungen der Erfindung jeweils für sich oder in einer beliebigen technisch sinnvollen Kombination auch untereinander jeweils mit dem Gegenstand des Anspruchs 1 kombinierbar sind.

Abwandlungen und Ausgestaltungen der Erfindung sowie weitere Vorteile und Einzelheiten der Erfindung lassen sich der nachfolgenden gegenständlichen Beschreibung und den Zeichnungen entnehmen. In den schematischen Figuren zeigen:
- Fig. 1: ein erfindungsgemäßes Terrarium,
- Fig. 2: das erfindungsgemäße Terrarium aus Fig. 1 in einer Vorderansicht,
- Fig. 3: eine Schnittdarstellung des erfindungsgemäßen Terrariums gemäß I-I in Fig. 2
- Fig. 4: eine Schnittdarstellung des erfindungsgemäßen Terrariums gemäß II-II in Fig. 3
- Fig. 4a: eine Detailvergrößerung des Ausschnitts III aus Fig. 4

Gleich oder ähnlich wirkende Teile sind - sofern dienlich - mit identischen Bezugsziffern versehen.

Einzelne technische Merkmale der nachbeschriebenen Ausführungsbeispiele können auch in Kombination mit vorbeschriebenen Ausführungsbeispielen sowie den Merkmalen der unabhängigen Ansprüche und etwaiger weiterer Ansprüche zu erfindungsgemäßen Gegenständen kombiniert werden.

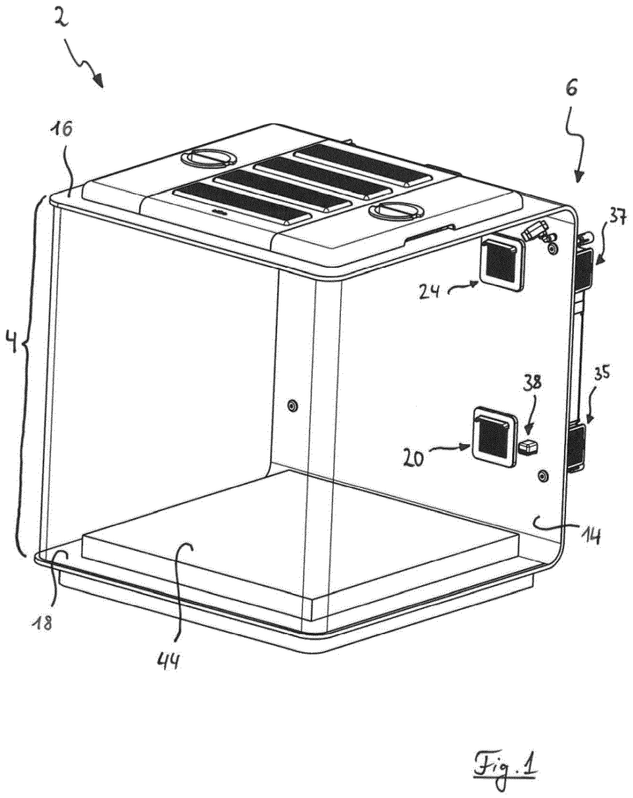

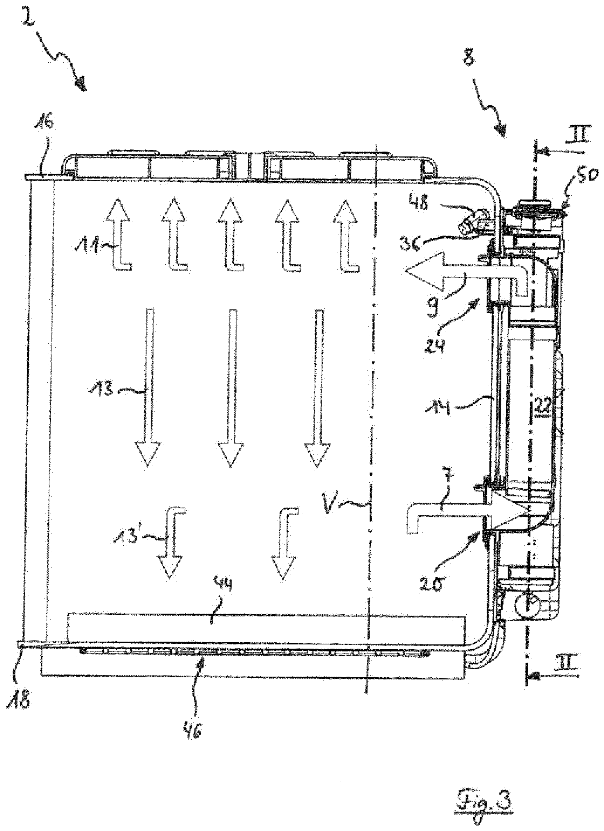

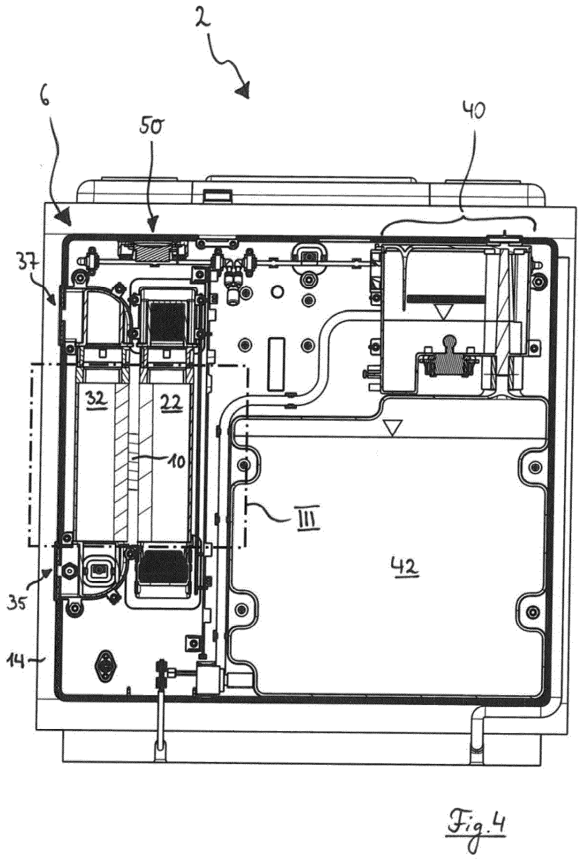

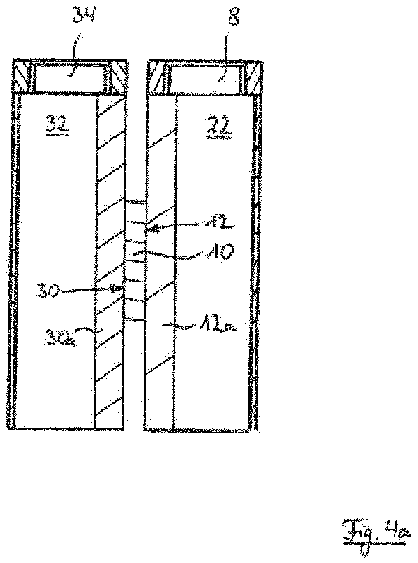

Die Figuren 1 bis 4a zeigen verschiedene (Teil-)Ansichten eines erfindungsgemä-ßen Terrariums 2 mit einem im Wesentlichen kubisch geformten Behältnis 4, das als Lebensraum insbesondere für ektotherme Tiere ausgebildet ist. Der Lebensraum wird von drei benachbarten transparenten Seitenwänden, einer Rückwand 14, einem Deckel 16 sowie einem Boden 18 begrenzt. Das Terrarium 2 ist mit einer Klimatisierungsvorrichtung 6 ausgestattet, die in der gezeigten Ausführungsform größtenteils hinter der Rückwand 14 angeordnet ist und in den Fig. 3, 4 und 4a genauer dargestellt ist.

Die Klimatisierungsvorrichtung 6 weist eine Strömungsmaschine 8 (z.B. einen Lüfter) und einen elektrothermischen Wandler 10 auf, beispielsweise in Form eines Peltier-Elements. Mittels des durch die Strömungsmaschine 8 erzeugten Druckunterschieds saugt die die Klimatisierungsvorrichtung 6 über eine in der Rückwand 14 eingelassene Einlassöffnung 20 Luft aus dem Behältnis 4 in einen Luftkanal 22 an. Die angesaugte Luft 7 wird innerhalb des Luftkanals 22 an einer ersten Seite 12 des elektrothermischen Wandlers 10 entlanggeführt und durch diesen konditioniert, d.h. je nach Betriebsmodus (Heiz- oder Kühlmodus) erhitzt oder gekühlt. Die konditionierte Luft 9 wird über eine in der Rückwand eingelassene Auslassöffnung 24 zurück in das Behältnis 4 befördert. In dem Behältnis 4 steigt wärmere Luft 11 auf, während kältere Luft 13, 13' absinkt. Es entsteht ein Temperaturgradient entlang einer Vertikalen V.

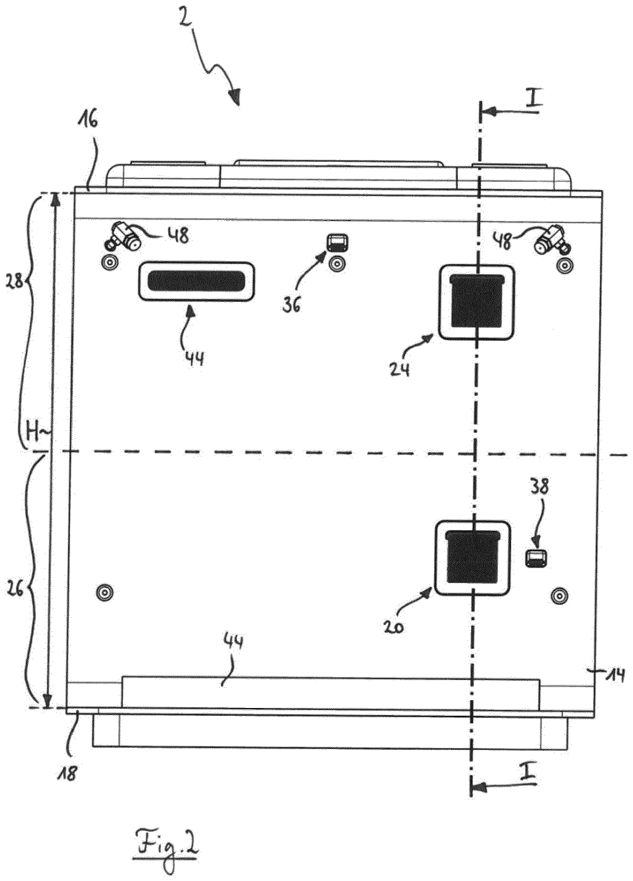

Wie aus Fig. 2 ersichtlich ist, ist die Einlassöffnung 20 dazu in einem unteren Bereich 26 und die Auslassöffnung 24 in einem oberen Bereich 28 des Behältnisses 4 angeordnet. In der vorliegenden Ausführungsform umfasst der untere Bereich 26 des Behältnisses 4 die untere Hälfte des Behältnisses 4 bezogen auf eine Gesamthöhe H des Behältnisses 4. Ferner umfasst der obere Bereich 28 die obere Hälfte des Behältnisses bezogen auf dessen Gesamthöhe H. Bei alternativen Ausführungsformen der Erfindung, wie Terrarien mit Wasseranteil oder Aquaterrarien, können der untere Bereich 26 und der obere Bereich 28 nicht auf die Gesamthöhe H des Behältnisses 4, sondern auf eine um einen Wasserspiegel innerhalb des Behältnisses 4 verkürzte Höhe, z.B. eine Luftraumhöhe, bezogen werden.

Wie in Fig. 4a zu sehen ist, ist in der vorliegenden Ausführungsform die erste Seite 12 mit einem in den Luftkanal 22 hineinragenden Kühlkörper 12a in Form eines thermisch leitenden Aufsatzes mit Kühlrippen ausgestattet. Dieser Kühlkörper 12a bildet einen Teil der Wandung des Luftkanals 22 aus, wobei seine zum elektrothermischen Wandler 10 hin gewandte Seite den Luftkanal 22 abschließt. Dies ermöglicht eine effiziente Konditionierung der Luft bei wenig benötigtem Bauraum.

Im Kühlmodus, d.h. wenn der elektrothermische Wandler 10 derart mit Strom durchflossen wird, dass die erste Seite 12 kalt wird, entsteht auf einer zweiten Seite 30 des elektrothermischen Wandlers 10, die dessen erster Seite 12 gegenüberliegt, Wärme. Zur verbesserten Abfuhr dieser Wärme weist die Klimatisierungsvorrichtung 6 einen Wärmeabfuhrluftkanal 32 auf, der außerhalb des Behältnisses 4 verläuft. Die zweite Seite 30 des elektrothermischen Wandlers 10 bildet dabei einen Teil der Wandung des Wärmeabfuhrluftkanals 32 aus. Die zweite Seite 30 weist einen Abluft-Kühlkörper 30a in Form eines Aufsatzes mit Kühlrippen auf, der in den Wärmeabfuhrluftkanal 32 hineinragt. Eine Abluft-Strömungsmaschine 34, zum Beispiel in Form eines Lüfters, erzeugt einen Abluftstrom, der die Wärmeabfuhr unterstützt. Eine Abluft-Einlassöffnung 35 und eine Abluft-Auslassöffnung 37 des Wärmeabluftkanals 32 sind nicht zum Behältnis 4 hin offen, sondern saugen Luft aus der Umgebung an und geben diese wieder an die Umgebung ab.

Der in der vorliegenden Ausführungsform im Wesentlichen plattenförmig ausgebildete elektrothermische Wandler 10 ist rechtwinklig zur Rückwand 14 angeordnet, die eine nächstliegende Wand des Terrariums 2 darstellt. Dadurch sind weder die erste Seite 12 noch die zweite Seite 30 des elektrothermischen Wandlers 10 direkt auf die Rückwand 14 gerichtet, so dass diese weder im Heizmodus durch die erste Seite 12 (Heizwärme) noch im Kühlmodus durch die zweite Seite 30 (Abwärme) zu stark erwärmt wird. Vielmehr wird durch die spezielle Anordnung des elektrothermischen Wandlers 10 zwischen dem Luftkanal 22 und dem Wärmeabfuhrluftkanal 32, die zumindest in ihren jeweiligen Abschnitten, in denen ein Teil ihrer Wandung von dem elektrothermischen Wandler 10 ausgebildet wird, parallel zueinander verlaufen, sowohl die Kälte als auch die Wärme in jeglichem Betriebsmodus optimal abtransportiert. Dies wird durch die verwendeten Kühlkörper 12a, 30 noch weiter verstärkt.

Das Terrarium 2 weist eine in den Zeichnungen nicht näher dargestellte Steuer- und Regelungseinheit auf, die mit einem ersten Sensor 36 sowie einem zweiten Sensor 38 datenübertragend verbunden ist. Der erste Sensor 36 ist in einem oberen Bereich 28 des Behältnisses angeordnet, während der zweite Sensor 38 in einem unteren Bereich 26 des Behältnisses 4 angeordnet ist. Beide Sensoren 36, 38 sind als 2-in-1-Kombisensoren zur Messung sowohl der Temperatur als auch der Luftfeuchtigkeit ausgebildet. Dadurch kann das Klima im Terrarium 2 in höherem Umfang reguliert und so das natürliche Habitat der zu haltenden Art noch besser nachgebildet werden. Die Steuer- und Regelungseinheit regelt die Strömungsmaschine 8 sowie den thermoelektrischen Wandler 6, wobei der Steuer- und Regelungseinheit ein Soll-Wert oder ein Soll-Wertebereich vorgegeben werden kann. Als Entscheidungskriterium über eine Ansteuerung des elektrothermische Wandlers 6 und/oder der Strömungsmaschine 8 kann ein Soll-Ist-Vergleich der vorgegeben Soll-Werte mit innerhalb des Behältnisses 4 durch zumindest einen der Sensoren 36, 38 aufgezeichneten Ist-Werten dienen.

In der vorliegenden Ausführungsform regelt die Steuer- und Regelungseinheit ferner einen Vernebler 40, mit dem Wasser aus einem Wasserreservoir 42 vernebelbar und zur Erhöhung der Luftfeuchtigkeit über eine Vernebleröffnung 44 in das Behältnis 4 abgebbar ist.

Zur optimalen und bequemen Einstellung des Klimas innerhalb des Terrariums 2 ist die Steuer- und Regelungseinheit derart eingerichtet, dass in Abhängigkeit des zu haltenden Tieres eine vorprogrammierte Parameterkonfiguration von klimataspezifischen Soll-Werten auswählbar ist, die zumindest eine Soll-Temperatur umfasst. Auf diese Weise kann beispielsweise mit einem Smartphone oder einer anderen Datenverarbeitungsanlage via App das für die jeweilige Tierart nachzubildende Biom ausgewählt werden. In der Steuer- und Regelungseinheit ist dem ausgewählten Biom eine Parameterkonfiguration zugewiesen, so dass die Steuer- und Regelungseinheit die Klimatisierungsvorrichtung 6 ansteuert und so die klimatischen Bedingungen im Terrarium 2 optimal das natürliche Habitat des zu haltenden Tieres anpassen kann.

Die Steuer- und Regelungseinheit kann auch mit weiteren Systemen der Klimatisierungsvorrichtung 6 kombiniert werden. In der gezeigten Ausführungsform regelt die Steuer- und Regelungseinheit eine mit einer Bodenplatte 44 des Behältnisses thermisch verbundene Bodenheizung 46 zur unterstützenden Temperaturregulierung, ein Beregnungssystem, das zwei im oberen Bereich 28 des Behälters 4 angeordnete Beregnungsdüsen 48 umfasst, sowie einen Lautsprecher 50 zur Imitation einer natürlichen akustischen Umgebung.

## Patentansprüche

1. (Aqua-)Terrarium (2), insbesondere für ektotherme Tiere, mit einem als Lebensraum dienenden Behältnis (4) und mit einer Klimatisierungsvorrichtung (6) umfassend eine Strömungsmaschine (8) und einen elektrothermischen Wandler (10), wobei die Strömungsmaschine (8) und/oder der elektrothermische Wandler (10) außerhalb des Behältnisses (4) angeordnet ist,
**dadurch gekennzeichnet, dass** die Klimatisierungsvorrichtung (6) dazu eingerichtet ist Luft aus dem Behältnis (4) anzusaugen, die angesaugte Luft (7) durch Entlangführen an einer ersten Seite (12) des elektrothermischen Wandlers (10) zu konditionieren und die konditionierte Luft (9) zurück in das Behältnis (4) zu befördern.

2. (Aqua-)Terrarium (2) nach Anspruch 1, **dadurch gekennzeichnet, dass** zumindest die erste Seite (12) des elektrothermischen Wandlers (10) rechtwinklig zu einer nächstliegenden Wand des Behältnisses (4) angeordnet ist.

3. (Aqua-)Terrarium (2) nach Anspruch 1 oder 2, **dadurch gekennzeichnet, dass** die Klimatisierungsvorrichtung (6) einen Luftkanal (22) aufweist, der zumindest teilweise außerhalb des Behältnisses (4) verläuft, wobei die erste Seite (12) des elektrothermischen Wandlers (10) oder ein mit der ersten Seite (12) thermisch leitend verbundener Kühlkörper (12a) einen Teil der Wandung des Luftkanals (22) ausbildet.

4. (Aqua-)Terrarium (2) nach einem der Ansprüche 1 bis 3, **dadurch gekennzeichnet, dass** die Klimatisierungsvorrichtung zum Behältnis (4) hin eine Einlassöffnung (20) und eine Auslassöffnung (24) aufweist, wobei die Einlassöffnung (20) in einem unteren Bereich (26) des Behältnisses (4) und die Auslassöffnung (24) in einem oberen Bereich (28) des Behältnisses (4) angeordnet ist.

5. (Aqua-)Terrarium (2) nach einem der Ansprüche 1 bis 4, **dadurch gekennzeichnet, dass** die Klimatisierungsvorrichtung (6) einen Wärmeabfuhrluftkanal (32) aufweist, der außerhalb des Behältnisses (4) verläuft, wobei eine zweite Seite (30) des elektrothermischen Wandlers (10), die dessen erster Seite (12) gegenüberliegt, oder ein mit der zweiten Seite (30) thermisch leitend verbundener Abluft-Kühlkörper (30a) einen Teil der Wandung des Abluftkanals (32) ausbildet.

6. (Aqua-)Terrarium (2) nach einem der Ansprüche 1 bis 5, **gekennzeichnet durch** eine Steuer- und Regelungseinheit, die mit zumindest einem Temperatursensor datenübertragend verbunden ist, und dazu eingerichtet ist die Strömungsmaschine (8) und/oder den thermoelektrischen Wandler (6) zu regeln.

7. (Aqua-)Terrarium (2) nach Anspruch 6, **dadurch gekennzeichnet, dass** die Steuer- und Regelungseinheit mit zumindest einem Luftfeuchtigkeitssensor datenübertragend verbunden ist und dazu eingerichtet ist einen Vernebler (40) der Klimatisierungsvorrichtung (6) zu regeln, insbesondere einen Ultraschall-Vernebler, mit dem Wasser aus einem Wasserreservoir (42) der Klimatisierungsvorrichtung (6) vernebelbar und zur Erhöhung der Luftfeuchtigkeit in das Behältnis (4) abgebbar ist.

8. (Aqua-)Terrarium (2) nach Anspruch 6 oder 7, **dadurch gekennzeichnet, dass** die Steuer- und Regelungseinheit zumindest mit einem ersten Sensor (36), der zumindest als Temperatursensor ausgebildet ist, und einem zweiten Sensor (38), der zumindest als Temperatursensor ausgebildet ist, datenübertragend verbunden ist, wobei der erste Sensor (36) in einem oberen Bereich (28) des Behältnisses (4) angeordnet ist und der zweite Sensor (38) in einem unteren Bereich (26) des Behältnisses (4) angeordnet ist.

9. (Aqua-)Terrarium (2) nach einem der Ansprüche 6 bis 8, **dadurch gekennzeichnet, dass** die Steuer- und Regelungseinheit derart eingerichtet ist, dass insbesondere in Abhängigkeit des zu haltenden Tieres eine vorprogrammierte Parameterkonfiguration von klimataspezifischen Soll-Werten auswählbar ist, die zumindest eine Soll-Temperatur bzw. einen Soll-Temperaturbereich umfasst.

## Claims

1. (Aqua)terrarium (2), in particular for ectothermic animals, having a container (4) which serves as a habitat and having a climate control device (6) which comprises a flow machine (8) and an electrothermal converter (10), the flow machine (8) and/or the electrothermal converter (10) being arranged outside the container (4),
**characterized in that** the climate control device (6) is designed to suck air from the container (4),
to condition the sucked-in air (7) by guiding it along a first side (12) of the electrothermal converter (10) and to convey the conditioned air (9) back into the container (4).

2. (Aqua)terrarium (2) according to claim 1, **characterized in that** at least the first side (12) of the electrothermal converter (10) is arranged at a right angle to a closest wall of the container (4).

3. (Aqua)terrarium (2) according to claim 1 or 2, **characterized in that** the climate control device (6) has an air channel (22) which extends, at least partly, outside the container (4), the first side (12) of the electrothermal converter (10) or a cooling body (12a) which is thermally conductively connected to the first side (12) forming part of the wall of the air channel (22).

4. (Aqua)terrarium (2) according to any of claims 1 to 3, **characterized in that** the climate control device has an inlet opening (20) and an outlet opening (24) at the container (4), the inlet opening (20) being arranged in a lower region (26) of the container (4) and the outlet opening (24) being arranged in an upper region (28) of the container (4).

5. (Aqua)terrarium (2) according to any of claims 1 to 4, **characterized in that** the climate control device (6) has a heat dissipation air channel (32) which extends outside the container (4), a second side (30) of the electrothermal converter (10), which is opposite its first side (12), or an exhaust air cooling body (30a) which is thermally conductively connected to the second side (30) forming part of the wall of the exhaust air channel (32).

6. (Aqua)terrarium (2) according to any of claims 1 to 5, **characterized by** an open-loop and closed-loop control unit which is connected to at least one temperature sensor in a data-transmitting manner and is designed to control the flow machine (8) and/or the thermoelectric converter (6) in a closed loop.

7. (Aqua)terrarium (2) according to claim 6, **characterized in that** the open-loop and closed-loop control unit is connected to at least one humidity sensor in a data-transmitting manner and is designed to control a mister (40) of the climate control device (6) in a closed loop,
in particular an ultrasonic mister, by means of which water from a water reservoir (42) of the climate control device (6) can be misted and released into the container (4) to increase the humidity.

8. (Aqua)terrarium (2) according to claim 6 or 7, **characterized in that** the open-loop and closed-loop control unit is connected to at least a first sensor (36) which is designed at least as a temperature sensor and to a second sensor (38) which is designed at least as a temperature sensor in a data-transmitting manner, the first sensor (36) being arranged in an upper region (28) of the container (4) and the second sensor (38) being arranged in a lower region (26) of the container (4).

9. (Aqua)terrarium (2) according to any of claims 6 to 8, **characterized in that** the open-loop and closed-loop control unit is designed such that, in particular depending on the animal to be kept, a pre-programmed parameter configuration of climate-specific target values can be selected, which comprises at least one target temperature or a target temperature range.

## Revendications

1. (Aqua)terrarium (2), en particulier pour des animaux ectothermes, comportant un récipient (4) servant d'espace de vie et un dispositif de climatisation (6) comprenant une turbomachine (8) et un convertisseur électrothermique (10), la turbomachine (8) et/ou le convertisseur électrothermique (10) étant disposés à l'extérieur du récipient (4),
**caractérisé en ce que** le dispositif de climatisation (6) est conçu pour aspirer de l'air à partir du récipient (4),
pour conditionner l'air aspiré (7) en le guidant le long d'un premier côté (12) du convertisseur électrothermique (10) et pour renvoyer l'air conditionné (9) dans le récipient (4).

2. (Aqua)terrarium (2) selon la revendication 1, **caractérisé en ce qu'au** moins le premier côté (12) du convertisseur électrothermique (10) est disposé perpendiculairement à une paroi la plus proche du récipient (4).

3. (Aqua)terrarium (2) selon la revendication 1 ou 2, **caractérisé en ce que** le dispositif de climatisation (6) présente un canal à air (22) qui s'étend au moins partiellement à l'extérieur du récipient (4), le premier côté (12) du convertisseur électrothermique (10) ou un corps de refroidissement (12a) relié de manière thermiquement conductrice au premier côté (12) formant une partie de la paroi du canal à air (22).

4. (Aqua)terrarium (2) selon l'une des revendications 1 à 3, **caractérisé en ce que** le dispositif de climatisation présente, vers le récipient (4), une ouverture d'entrée (20) et une ouverture de sortie (24), l'ouverture d'entrée (20) étant disposée dans une zone inférieure (26) du récipient (4) et l'ouverture de sortie (24) dans une zone supérieure (28) du récipient (4).

5. (Aqua)terrarium (2) selon l'une des revendications 1 à 4, **caractérisé en ce que** le dispositif de climatisation (6) présente un canal d'évacuation d'air chaud (32) qui s'étend à l'extérieur du récipient (4), un second côté (30) du convertisseur électrothermique (10), qui est opposé à son premier côté (12), ou un corps de refroidissement d'air évacué (30a) relié de manière thermiquement conductrice au second côté (30) formant une partie de la paroi du canal d'évacuation d'air (32).

6. (Aqua)terrarium (2) selon l'une des revendications 1 à 5, **caractérisé par** une unité de commande et de régulation qui est reliée à au moins un capteur de température de manière à transmettre des données et qui est configurée pour réguler la turbomachine (8) et/ou le convertisseur thermoélectrique (6).

7. (Aqua)terrarium (2) selon la revendication 6, **caractérisé en ce que** l'unité de commande et de régulation est reliée à au moins un capteur d'humidité de l'air de manière à transmettre des données et configurée pour réguler un nébuliseur (40) du dispositif de climatisation (6),
en particulier un nébuliseur à ultrasons, avec lequel de l'eau provenant d'un réservoir d'eau (42) du dispositif de climatisation (6) peut être nébulisée et peut être distribuée dans le récipient (4) pour l'augmentation de l'humidité de l'air.

8. (Aqua)terrarium (2) selon la revendication 6 ou 7, **caractérisé en ce que** l'unité de commande et de régulation est reliée de manière à transmettre des données au moins à un premier capteur (36), lequel capteur est conçu au moins comme un capteur de température, et à un second capteur (38), lequel est conçu au moins comme un capteur de température, le premier capteur (36) étant disposé dans une zone supérieure (28) du récipient (4) et le second capteur (38) étant disposé dans une zone inférieure (26) du récipient (4).

9. (Aqua)terrarium (2) selon l'une des revendications 6 à 8, **caractérisé en ce que** l'unité de commande et de régulation est configurée de telle sorte qu'une configuration de paramètres préprogrammée de valeurs de consigne spécifiques au climat, qui comprend au moins une température de consigne ou une plage de températures de consigne, peut être sélectionnée en particulier en fonction de l'animal à détenir.
